(19) Europäisches Patentamt
European Patent Office
Office européen des brevets

(11) Numéro de publication: **0 472 671 B1**

# (12) FASCICULE DE BREVET EUROPEEN

(45) Date de publication de fascicule du brevet: **15.03.95**

(51) Int. Cl.[6]: **C12Q 1/56**, C07K 5/08, C07K 5/10, G01N 33/68

(21) Numéro de dépôt: **91902116.2**

(22) Date de dépôt: **31.12.90**

(86) Numéro de dépôt internationale : **PCT/FR90/00974**

(87) Numéro de publication internationale : **WO 91/12338 (22.08.91 91/19)**

## (54) SUBSTRATS PEPTIDIOUES POUR IDENTIFICATION DU FACTEUR Xa.

(30) Priorité: **19.02.90 FR 9001965**

(43) Date de publication de la demande: **04.03.92 Bulletin 92/10**

(45) Mention de la délivrance du brevet: **15.03.95 Bulletin 95/11**

(84) Etats contractants désignés: **DE**

(56) Documents cités:
**EP-A- 110 306**
**EP-A- 280 610**
**WO-A-81/02294**
**WO-A-87/00056**
**WO-A-87/05608**

(73) Titulaire: **SERBIO**
**Parc d'Activités Economiques,**
**Parispace 3,**
**125, avenue Louis Roche**
**F-92230 Gennevilliers (FR)**

(72) Inventeur: **OUENTIN, Gérard**
**145, rue des Renouillers**
**F-92700 Colombes (FR)**
Inventeur: **MARTINOLI, Jean-Luc**
**2, rue du Tremble**
**F-92390 Villeneuve-la-Garenne (FR)**

(74) Mandataire: **Clisci, Serge et al**
**S.A. FEDIT-LORIOT & AUTRES**
**38, avenue Hoche**
**F-75008 Paris (FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

## DOMAINE DE L'INVENTION

La présente invention a trait en tant que produits industriels nouveaux à des composés tri- et tétrapeptidiques. Ces nouveaux produits sont particulièrement utiles en tant que substrats pour l'identification et le dosage du Facteur Xa (i.e. Facteur X activé) intervenant dans les mécanismes de l'hémostase. L'invention concerne également le procédé de préparation de ces nouveaux produits ainsi que le procédé de dosage et/ou d'identification du Facteur Xa au moyen desdits produits.

## ART ANTERIEUR

On sait que les enzymes de la classe E.C. 3.4.21 [telle que définie dans l'ouvrage "Enzyme Nomenclature", Elsevier Scientific Publishing Company, Amsterdam 1973, pages 238 et suivantes (ancienne nomenclature : classe E.C. 3.4.4.)] sont des substances qui scindent les liaisons amides du squelette protéique ou peptidique du côté du groupe carboxyle des restes Arg, Lys, Orn et His. Il s'agit là d'un mécanisme de clivage bien connu de l'homme de métier et qui est amplement exemplifié dans les documents antérieurs cités ci-après.

Les substrats d'enzymes de ladite classe actuellement utilisés sont des composés essentiellement tri- ou tétrapeptidiques dont l'extrémité N-terminale est généralement substituée par un groupe de blocage tel que benzoyle, benzyloxycarbonyle, t-butyloxycarbonyle, t-amyloxycarbonyle, tosyle, acétyle ou analogue, et dont l'extrémité CO-terminale est amidifiée par un groupe aminé pouvant être un reste radioactif ou un reste capable de conférer, avant ou (mieux) après clivage, une coloration ou une fluorescence, notamment un groupe p-nitroanilino. Voir à cet effet, les documents de brevet suivants FR-A-2 372 798, EP-A-0 004 256, US-A 4 508 644, US-A-4 448 715, FR-A-2 471 411, FR-A-2 317 280 et FR-A-2 459 226.

Il se trouve que les dérivés peptidiques dans les documents de brevets précités ont peu d'affinité pour l'eau. Ils sont peu solubles ou peu dispersables dans l'eau; par suite il est quelquefois nécessaire de leur adjoindre un solvant organique pour pouvoir les utiliser. Les systèmes comprenant des solvants mixtes ne sont guère compatibles, d'une manière générale, avec les milieux biologiques : ils entraînent soit une diminution de l'activité du substrat, soit encore, dans certains cas, une déterioration de l'enzyme que l'on veut doser. De plus, la faible affinité pour l'eau de ces substrats entraîne une diminution de la sensibilité des méthodes de dosage des enzymes.

Pour améliorer l'hydrosolubilité des substrats enzymatiques, on connaît de EP-A-0 025 190 une première solution technique qui consiste à substituer sur ladite extrémité N-terminale un reste de polyéthylèneglycol monoéthérifié par un groupe alkyle, par exemple $Me\text{-}O(CH_2CH_2O)_x\text{-}CO$ (où x est un nombre entier tel que le reste de polyéthylèneglycol ait un poids moléculaire moyen de 600 environ), le reste aminoacide comportant l'extrémité CO-terminale de la chaîne peptidique étant différent des restes Arg et Lys qui interviennent selon l'invention comme on le verra ci-après ; et on connait de de EP-A-0 280 610 (de la Demanderesse) une autre solution technique qui consiste à fixer sur l'extrémité N-terminale d'un dipeptide un reste alkyloxycarbonylméthylènecarbonyle, tel que le reste méthoxymalonyle (i.e. $Me\text{-}O\text{-}CO\text{-}CH_2\text{-}CO$ ; en abrégé MM).

Par ailleurs selon le document EP-A-0 280 610 précité les substrats dipeptidiques comportant ledit reste méthoxymalonyle sur ladite extrémité N-terminale sont, d'une manière générale présentés comme étant plus sensibles vis-à-vis des enzymes de la classe E.C. 3.4.21 que les substrats tri- et tétrapeptidiques comportant ledit reste méthoxymalonyle. Toutefois ces substrats dipeptidiques selon EP-A-0 280 610 ne sont pas particulièrement spécifiques vis-à-vis du Facteur Xa et ne permettent pas un dosage efficace dudit Facteur Xa.

On sait que le Facteur Xa, qui fait partie de la sous-classe E.C. 3.4.21.6, est un des éléments importants intervenant dans les mécanismes de l'hémostase. L'activation du Facteur X conduit à la formation du Facteur Xa, qui est une sérine protéase responsable de la transformation de la prothrombine en thrombine. Il est donc très intéressant, du point de vue du diagnostic, de disposer de substrats peptidiques spécifiques du Facteur Xa et utilisables selon une technique de détermination simple avant tout accident thromboembolique néfaste.

Pour la détermination du Facteur Xa on sait que l'on a déjà préconisé des substrats tri- et tétrapeptidiques. Parmi les substrats tripeptidiques qui ont été proposés à cet effet on connait en particulier :

- de EP-A-0 004 256 précité, les
  Cbo-L-Pyr-Gly-L-Arg-pNA,
  H-L-Pyr-Gly-L-Arg-pNA

et leurs sels d'addition d'acide;

- de EP-A-0 110 306, les
  Z-D-Leu-Gly-L-Arg-pNA
  (présenté comme illustrant l'art antérieur ; voir tableau VIII page 32 dudit document EP-A-0 110 306),

  Z-D-Leu-Gly-L-Arg(2-OMe)pNA
  Z-D-Leu-Gly-L-Arg(2-CONHMe)pNA
  Z-D-Leu-Gly-L-Arg(2-COOBu)pNA,

  H-D-Lys(Z)-Gly-L-Arg(2-Z)pNA,
  Tos-Gly-L-Pro-L-Arg(2-CONHiPr)pNA,
  et leurs sels d'addition ; et,
- de US-A-4 440 678 et US-A-4 480 030 (qui correspondent à EP-A-0 034 122), les composes de formule

```
X1-D-NH-CH-CO-N——CH-CO-L-Arg-X5      (Io)
          |     |  |
          X2    X3 X4
```

dans laquelle

$X^1$ est alcanoyle en $C_2$-$C_8$, ω-aminoalcanoyle en $C_2$-$C_8$, phénylalcanoyle (où le fragment alcanoyle est en $C_2$-$C_4$ et le fragment phényle peut être substitué en position para par $NH_2$), cyclohexylcarbonyle (pouvant être substitué en position 4 par MeNH), benzoyle (pouvant être substitué en position ortho ou para), alkoxycarbonyle (où le fragment alkoxy est en $C_1$-$C_8$), benzyloxycarbonyle (où le fragment benzyloxy peut être substitué en position para par MeO, Me ou Cl), alcanesulfonyle en $C_1$-$C_4$, phénylsulfonyle (où le fragment phényle peut être substitué en position para par Me), α- ou β-napthylsulfonyle,

$X^2$ est alkyle en $C_1$-$C_6$ à chaîne hydrocarbonée linéaire ou ramifiée, hydroxyalkyle en $C_1$-$C_2$, alkoxyalkyle (où le fragment alkoxy est en $C_1$-$C_4$ et le fragment alkyle en $C_1$-$C_2$), ω-carboxyalkyle ou ω-alkoxycarbonylalkyle (où les fragments alkyle sont en $C_1$-$C_3$ et le fragment alkoxy est en $C_1$-$C_4$), ω-benzyloxycarbonylalkyle (où le fragment alkyle est en $C_1$-$C_3$), cyclohexyle, cyclohexylméthyle, 4-hydroxy-cyclohexylméthyle, phényle, benzyle, 4-hydroxybenzyle ou imidazol-4-yl-méthyle, avec la condition que $X^1$ est différent du groupe benzyloxycarbonyle quand $X^2$ est isopropyle ou cyclohexyle,

$X^3$ est alkyle en $C_1$-$C_4$,

$X^4$ est H, Me ou Et,

$X^5$ est un groupe aminé comportant un groupe amino substitué par un reste aromatique ou hétérocyclique, clivable par hydrolyse enzymatique pour donner un compose H-$X^5$ coloré ou fluorescent détectable photométriquement, spectrophotométriquement, fluorophotométriquement, la quantité de H-$X^5$ libérée étant proportionnelle à la quantité d'enzyme provoquant le clivage du substrat peptidique ; et leurs sels d'addition d'acide.

Parmi les composés de formule Io, qui ont été proposés pour la détermination du Facteur Xa, on peut notamment citer les tripeptides :

Cbo-D-CHG-Gly-L-Arg-pNA,
Cbo-D-CHT-MeGly-L-Arg-pNA,
Cbo-D-CHA-MeGly-L-Arg-pNA,
Cbo-D-Nle-MeGly-L-Arg-pNA,
$MeSO_2$-D-CHA-Gly-L-Arg-pNA,
Boc-D-AOA-Gly-L-Arg-pNA
et leurs sels d'addition d'acide.

Parmi les composés tétrapeptidiques qui ont été décrits comme substrats utiles dans la détermination du Facteur Xa, on connaît en particulier :

- le composé S-2222 (commercialisé par la société dite Kabi Diagnostica, Stockholm, Suède) qui répond à la formule :
  BZ-L-Ile-Glu-Gly-L-Arg-pNA, HCl ;

- de CH-A- 637 627 (correspondant à FR-A-2 372 798 précité) les composés
  Bz-L-Ile-Glu(OMe)-Gly-L-Arg-pNA,
  Bz-L-Ile-Asp(Mor)-Gly-L-Arg-pNA
  et leurs sels d'addition d'acide;
- de EP-A-0 010 306 précité, le
  Bz-L-Ile-L-Glu(OMe)-Gly-L-Arg-(2-CONHMe)pNA et ses sels d'addition d'acide.

L'art antérieur relatif aux substrats tri- ou tétrapeptidiques préconisés pour la détermination du Facteur Xa enseigne que la spécificité est fonction (i) de la nature de la chaîne peptidique, (ii) de la structure du groupe de blocage de l'extrémité N-terminale, et (iii) du choix du groupe révélateur clivable de l'extrémité CO-terminale de ladite chaîne.

**OBJET DE L'INVENTION**

Suivant l'invention on préconise une nouvelle solution technique qui se différencie de l'art antérieur par le choix d'un groupe (i) bloquant l'extrémité N-terminale de la chaîne peptidique, et (ii) améliorant l'hydrosolubilité ou l'affinité pour l'eau des composés tri- ou tétrapeptidiques utiles dans la détermination du Facteur Xa.

Ainsi, selon un premier aspect de l'invention on préconise en tant que produits industriels nouveaux, des composés peptidiques qui sont caractérisés en ce qu'ils sont choisis parmi l'ensemble constitué par

(i) les tri- et tétrapeptides répondant à la formule

$$Q\text{-}A_1\text{-}A_2\text{-Gly-}A_4\text{-R} \quad (I)$$

dans laquelle

Q est un groupe bloquant l'extrémité N-terminale de la chaîne peptidique et qui est choisi parmi l'ensemble constitué par

(a) un reste oxymalonyle de formule $R_1$-O-CO-$CH_2$-CO, où $R_1$ est
- un groupe alkyle en $C_1$-$C_4$,
- un groupe phényle,
- un groupe phényle substitué par un ou plusieurs groupes Me, MeO, Cl, Br, F, $CF_3$ ;
- un groupe cycloalkyle en $C_3$-$C_6$,
- un groupe benzyle,
- un groupe benzyle substitué par un ou plusieurs groupes Me, MeO, Cl, Br, F, $CF_3$, ou
- un groupe cycloalkylméthyle où le fragment cycloalkyle est en $C_3$-$C_6$; et

(b) un reste de polyéthylèneglycol de formule $R_2$-O($CH_2CH_2O)_n$-CO, où $R_2$ est un groupe alkyle en $C_1$-$C_6$, phényle ou benzyle et n est un nombre entier ayant pour valeur 1 à 170,

$A_1$ est choisi parmi l'ensemble constitué par les groupes Leu, Ile, Nle, Nva, CHA, CHG, CHT, Phe, Ala et Lys où la fonction basique latérale du reste Lys est bloquée par un groupe convenable éliminant substantiellement le caractère basique de ladite fonction latérale,

$A_2$ est une simple liaison, Asp ou Glu, le groupe acide carboxylique latéral de Asp et Glu étant susceptible d'être estérifié ou amidifié,

$A_4$ est choisi parmi l'ensemble constitué par les groupes Arg et Lys,

R est un moyen de marquage clivable ; et

(ii) leurs sels d'addition.

Selon un second aspect de l'invention on préconise l'utilisation des composés de formule I et de leurs sels d'addition d'acide en tant que substrats enzymatiques pour la détermination du Facteur Xa.

On a en effet trouvé de façon surprenante que les tri- et tétrapeptides selon l'invention sont plus intéressants que les substrats tri- et tétrapeptidiques antérieurement connus dans le domaine de la détermination du Facteur Xa, soit par ce qu'ils sont plus actifs, soit par ce qu'ils sont plus sélectifs, soit encore par ce qu'ils sont plus hydrosolubles que lesdits substrats antérieurs.

Suivant un autre aspect de l'invention on préconise un procédé pour la préparation des composés de formule I et de leurs sels d'addition.

Suivant encore un autre aspect de l'invention on fournit un procédé de détermination du Facteur Xa au moyen des composés de formule I ou de leurs sels d'addition.

**ABREVIATIONS**

Dans la présente description, on a utilisé les abréviations suivantes par commodité :

- pour les restes d'aminoacide

Ala = α-alanyle
β-Ala = β-alanyle
AOA = α-aminooctanoyle
Arg = arginyle
Asp = α-aspartyle
CHA = 3-cyclohexylalanyle
CHG = α-cyclohexylglycyle
CHT = 3-(4-hydroxycyclohexyl)alanyle
Ile = isoleucyle
Leu = leucyle
Lys = lysyle
MeGly = N-méthylglycyle (ou sarcosyle)
Nle = norleucyle
Nva = norvalyle
Phe = phénylalanyle
Pro = prolyle
Pyr = pyroglutaminyle (ou 2-pyrrolidone-5-carbonyle)

- pour les autres abréviations :

Ac = acétyle
AcOH = acide acétique
Adoc = adamantyloxycarbonyle
Aoc = t-amyloxycarbonyle
Boc = t-butyloxycarbonyle
Bop = hexafluorophosphate de 1-(benzotriazolyl)oxy-tris(diméthylamino)phosphonium (autre nomenclature réactif de CASTRO) de formule

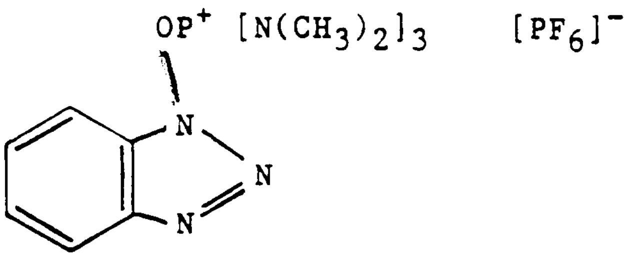

Bu = n-butyle
Bz = benzoyle
Bzl = benzyle
Cbo = carbobenzoxy
CCM = chromatographie sur couche mince
DCCI = dicyclohexylcarbodiimide
DIEA = diisopropyléthylamine
DMF = diméthylformamide
Et = éthyle
EM = éthyloxymalonyle (EtO-CO-$CH_2$-CO)
$Et_3N$ = triéthylamine
EtO = éthoxy
Fmoc = 9-fluorènylméthyloxycarbonyle
Foc = furfuryloxycarbonyle
HMPT = N,N,N',N',N'',N''-hexaméthylphosphorotriamide
HOBT = 1-hydroxybenzotriazole
HPLC = chromatographie liquide haute performance

| | | |
|---|---|---|
| H-TFA | = | acide trifluoroacétique (ou encore HTFA) |
| Iboc | = | isobornyloxycarbonyle |
| iBu | = | isobutyle |
| iPr | = | isopropyle |
| Me | = | méthyle |
| MeO | = | méthoxy |
| MeOH | = | méthanol |
| MM | = | méthyloxymalonyle (MeO-CO-$CH_2$-CO) |
| Mor | = | 1-morpholinyle |
| OD | = | densité optique |
| OSu | = | N-oxysuccinimide ou (2,4-dioxopyrrolidin-1-yl)oxy |
| PEG | = | polyéthylèneglycol |
| Ph | = | phényle |
| pH | = | cologarithme de la concentration en ions $H^+$ |
| Pi | = | 1-pipéridinyle |
| PM | = | poids moléculaire |
| pNA | = | p-nitroanilino ou (4-$NO_2$)$C_6H_4$NH |
| Pr | = | n-propyle |
| Py | = | 1-pyrrolidinyle |
| RT | = | température ambiante (15-20 °C) |
| tBu | = | t-butyle |
| THF | = | tétrahydrofuranne |
| Tos | = | p-toluènesulfonyle (ou tosyle) |
| TR | = | temps de rétention |
| Z | = | benzyloxycarbonyle |
| Z(p-Cl) | = | p-chlorobenzyloxycarbonyle |
| Z(p-OMe) | = | p-méthoxybenzyloxycarbonyle |

## DESCRIPTION DETAILLEE DE L'INVENTION

Comme indiqué plus haut le groupe Q est un groupe bloquant l'extrémité N-terminale de la chaîne peptidique $A_1$-$A_2$-Gly-$A_4$. Il représente un reste oxymalonyle de formule $R_1$-O-CO-$CH_2$-CO ou un reste de PEG de formule $R_2$-O($CH_2CH_2O$)$_n$-CO. Dans ledit reste d'oxymalonyle, $R_1$ peut notamment représenter

- un groupe alkyle en $C_1$-$C_4$, de préférence Me, Et, Pr, iPr, Bu ou tBu,
- un groupe phényle,
- un groupe tolyle, xylyle, 2-, 3- ou 4-méthoxyphényle 2,4-, 2,6-, 3,4- ou 3,5-diméthoxyphényle, 4-chlorophényle, 4-fluorophényle, 3,4-, 3,5- ou 2,6-dichlorophényle, 3-trifluorométhylphényle,
- un groupe cyclopropyle, cyclopentyle ou cyclohexyle,
- un groupe benzyle,
- un groupe 2-, 3- ou 4-méthylbenzyle, 2-, 3- ou 4-méthoxybenzyle, 2,4-, 2,6-, 3,4- ou 3,5-diméthylbenzyle, 2,4-, 2,6-, 3,4- ou 3,5-diméthoxybenzyle, 2-, 3- ou 4-chlorobenzyle, 2-, 3- ou 4-fluorobenzyle, 2,4-, 2,6-, 3,4- ou 3,5-dichlorobenzyle, 3-trifluorométhylbenzyle, ou
- un groupe cyclopropyl-, cyclopentyl- ou cyclohexylméthyle.

Quand $R_1$ est un groupe phényle substitué ou benzyle substitué les substitutions seront avantageusement placées en position 2,3 et/ou 4. De façon encore plus avantageuse $R_1$ sera Me ou Et, pour conférer une plus grande affinité au composé peptidique vis-à-vis de l'eau; en d'autres termes, Q sera alors MM ou EM.

En ce qui concerne le fragment O($CH_2CH_2O$)$_n$ du reste de PEG, n sera, comme indiqué plus haut, un nombre entier ayant pour valeur 1 à 170. En particulier le PM moyen du fragment O($CH_2CH_2O$)$_n$ sera compris entre environ 60 (n = 1) et environ 7100 (n = 161). De façon avantageuse dans ledit reste de PEG $R_2$ sera Me, Et, Pr, iPr, Bu, tBu ou n-hexyle ; de façon encore plus avantageuse $R_2$ sera Me ou Et.

Comme indiqué ci-dessus, $A_1$ est un reste d'aminoacide choisi parmi l'ensemble constitué par Leu, Ile, Nle, Nva, CHA, CHG, CHT, Phe, Ala et Lys où la fonction basique latérale du reste Lys est bloquée par un groupe acide convenable [tel que Boc, Cbo, Z, Z(p-Cl), Z(p-OMe), Adoc, Aoc, Fmoc, Foc, Iboc]. Dans le cas du reste Lys un tel groupe acide permet d'éliminer pratiquement le caractère basique de la fonction basique latérale.

Le reste $A_1$ peut être de configuration L ou D, toutefois pour disposer d'une meilleure activité ou sensibilité, en ce qui concerne la détermination du Facteur Xa, on préfère plutôt que ledit reste $A_1$ soit de

configuration D.

C'est pourquoi $A_1$ sera avantageusement choisi parmi l'ensemble constitué par les groupes D-Leu, D-Ile, D-Nle, D-Nva, D-CHA, D-CHG, D-CHT, D-Phe, D-Ala et D-Lys où la fonction basique latérale de D-Lys est bloquée par un groupe acide convenable.

Les restes $A_1$ que l'on considère comme étant les plus intéressants selon l'invention sont D-Nle, D-Leu et D-CHA.

Comme indiqué plus haut, $A_2$ représente une simple liaison ou un reste Asp ou Glu, le groupe carboxy latéral de chaque reste Asp et Glu pouvant le cas échéant être estérifié [i.e. remplacement du groupe OH de la fonction COOH par un groupe $OR_3$ où $R_3$ est notamment alkyle en $C_1$-$C_4$ (par exemple Me, Et, iPr, Pr, Bu, tBu ou iBu), hydroxyalkyle en $C_1$-$C_4$ (de préférence $CH_2CH_2OH$), cycloalkyle en $C_3$-$C_6$ (tel que cyclopropyle, cyclopentyle ou mieux cyclohexyle) ou $\omega$-aminoalkyle où le fragment alkyle est en $C_2$-$C_4$ et où l'atome d'azote du fragment amino peut être monoalkylé ($NHR_4$), dialkylé ($NR_4R_5$) ou inclus dans un cycle ($NR_4R_5$ = groupe N-hétérocyclique)] ou amidifié [i.e. remplacement du groupe OH de la fonction COOH par un groupe $NH_2$, $NHR_4$ ou $NR_4R_5$ (où $R_4$ et $R_5$, identiques ou différents, représentent chacun un groupe alkyle en $C_1$-$C_4$, $R_4$ et $R_5$ considérés ensemble pouvant former avec l'atome d'azote, auquel ils sont liés, un groupe N-hétérocyclique de 5 à 7 sommets notamment choisi parmi l'ensemble constitué par les groupes pyrrolidino, pipéridino, morpholino, thiomorpholino, pipérazino, 4-(2-hydroxyéthyl)-pipérazino, 4-méthylpipérazino, 4-(4-chlorophényl)pipérazino, hexaméthylèneimino)].

Des exemples de restes Glu et Asp, où le groupe COOH latéral est estérifié ou amidifié, sont présentés dans les documents CH-A-637 627 et FR-A-2 372 798 précités, incorporés ici à titre de référence.

De façon avantageuse, lorsque $A_2$ représente un reste d'aminoacide, ledit reste sera de configuration L ; $A_2$ sera donc soit une simple liaison soit un reste Glu ou Asp notamment choisi parmi l'ensemble constitué par les groupes L-Glu, L-Asp, L-Glu(Mor), L-Glu(Py), L-Glu(Pi), L-Glu(OMe), L-Glu (OEt), L-Glu-(OtBu), L-Asp(Mor), L-Asp(Py), L-Asp(Pi), L-Asp(OMe), L-Asp(OEt) et L-Asp(OtBu).

De façon également avantageuse, $A_4$ représentera L-Arg ou L-Lys.

Le moyen de marquage R est bien connu dans la technique des dosages biologiques et microbiologiques, voir à cet effet l'art antérieur précité et notamment le document US-A-4 448 715. Ledit moyen de marquage sera de préférence choisi parmi l'ensemble des groupes aminés NH-R' qui (i) induisent une modification de coloration, (ii) induisent une modification de fluorescence ou (iii) comportent au moins un élément radioactif (par exemple un groupe anilino ou benzylamino marqué par un radioisotope $^{14}C$ ou $^{3}H$). Convient au but de l'invention tout groupe amino NH-R' donnant pendant ou après la réaction enzymatique un signal susceptible d'être amplifié pour être détecté (par exemple par mesure de la densité optique sous une longueur d'onde donnée, ou encore par mesure de la radioactivité). La quantité de produit H-R obtenu par clivage lors de l'hydrolyse enzymatique est proportionnelle à la quantité d'enzyme utilisée. Ladite quantité de H-R est déterminable photométriquement, spectrophotométriquement, fluorospectrophotométriquement ou électrochimiquement.

Le groupe R que l'on préfère suivant l'invention est un groupe chromogène, de façon typique un groupe nitrophénylamino (dans lequel le reste phényle est susceptible d'être substitué par un groupe COOH, F, Cl, Br, $CH_3$, $OCH_3$, CN, $CF_3$, et/ou $SO_3H$), ou un groupe fluorogène, de façon typique un groupe naphtylamino (dans lequel le reste naphtyle est susceptible d'être substitué par un groupe $OCH_3$, COOH, $SO_3H$ ou $CH_3$), et les groupes 4-méthylcoumaryl-7-amino, 4-trifluorométhylcoumaryl-7-amino et analogues.

Parmi les groupes aminés chromogènes et fluorogènes,qui conviennent suivant l'invention, on peut notamment citer les p-nitroanilino (en abrégé pNA), 2-carboxy-4-nitroanilino et 3-carboxy-4-nitroanilino, 2-halogéno-4-nitroanilino et 3-halogéno-4-nitroanilino (où l'halogène est F, Cl ou Br), 2-méthoxy-5-méthyl-4-nitroanilino, 2-hydroxysulfonyl-4-nitroanilino, 4-trifluorométhyl-2-nitroanilino, 4-trifluorométhyl-3-nitroanilino, 4-cyano-2-nitroanilino, 2-naphtylamino, 4-hydroxysulfonyl-1-naphtylamino, quinolylamino, nitroquinolylamino et analogues.

Le groupe R préféré suivant l'invention est un groupe chromogène à savoir, d'une part, pNA et, d'autre part, les groupes analogues, où le noyau phényle de pNA est substitué en position 2 ou 3 qui répondent à la formule

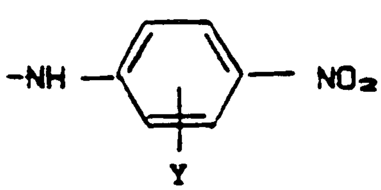

(II)

dans laquelle Y est Br, Cl, F, $CF_3$, COOH, COOW, $CONH_2$, CONHW, $CONW_2$, $CONH(CH_2)_mNMe_2$, OH ou OW, où W est un groupe alkyle en $C_3$-$C_6$, aryle en $C_6$-$C_{10}$, aralkyle en $C_7$-$C_{11}$ ou alicyclique en $C_3$-$C_8$ et m est un nombre entier ayant pour valeur 1 à 10.

De tels groupes de formule II où le noyau phényle du groupe pNA est substitué sont notamment décrits dans le document EP-A-0 110 306 précité.

Les sels d'addition suivant l'invention sont essentiellement des sels d'addition d'acide obtenus par réaction d'un composé de formule I avec un acide minéral ou organique.

Le meilleur mode de mise en oeuvre de l'invention consiste à utiliser comme substrat un composé tri- ou tétrapeptidique répondant à la formule

$Q$-$A_1$-$A_2$-Gly-$A_4$-pNA (III)

dans laquelle

- Q est MM, EM ou $R_2$-$O(CH_2CH_2O)_n$-CO où $R_2$ est Me, Et, Pr, iPr, Bu, iBu, ou n-hexyle et où n est un nombre entier compris entre 1 et environ 161,
- $A_1$ est choisi parmi l'ensemble constitué par D-Leu, D-Ile, D-Nle, D-Nva, D-CHA, D-CHG, D-CHT, D-Phe, D-Ala, D-Lys(Cbo) et D-Lys(Boc), les groupes $A_1$ préférés étant D-Nle, D-Leu et D-CHA,
- $A_2$ est choisi parmi l'ensemble constitué par une simple liaison et les groupes L-Glu et L-Asp où le groupe OH du reste acide carboxylique latéral desdits L-Glu et L-Asp peut être remplacé par OMe, OEt, OtBu, morpholino, pipéridino ou pyrrolidino,
- $A_4$ est choisi parmi l'ensemble constitué par L-Arg et L-Lys,

et ses sels d'addition d'acide.

De façon non limitative on a consigné dans les tableaux I et II ci-après un certain nombre de composés peptidiques (composés "Ex") selon l'invention. Par commodité, on a également mentionné dans le tableau I un peptide de référence qui a été commercialisé en tant que substrat spécifique du Facteur Xa pour comparaison.

Les composés peptidiques selon l'invention, qui se sont révélés être particulièrement intéressants sont notamment les suivants :

(a) MM-D-Leu-Gly-L-Arg-pNA,
(b) EM-D-Leu-Gly-L-Arg-pNA,
(c) $MeO(CH_2CH_2O)$-CO-D-Leu-Gly-L-Arg-pNA,
(d) $MeO(CH_2CH_2O)_2$-CO-D-Leu-Gly-L-Arg-pNA,
(e) $MeO(CH_2CH_2O)_3$-CO-D-Leu-Gly-L-Arg-pNA,
(f) $MeO(CH_2CH_2O)_7$-CO-D-Leu-Gly-L-Arg-pNA,
(g) MM-D-Nle-Gly-L-Arg-pNA,
(h) MM-D-Nle-L-Glu(Py)-Gly-L-Arg-pNA,
(i) MM-D-CHA-Gly-L-Arg-pNA,
(j) MM-D-Ile-L-Glu(OMe)-Gly-L-Arg-pNA,
(k) EM-D-Nle-Gly-L-Arg-pNA, et

(l) leurs sels d'addition d'acide.

**TABLEAU I**

$Q-A_1-A_2-Gly-A_4-pNA, HA$

| Produit | Q | $A_1$ | $A_2$ | $A_4$ | HA |
|---|---|---|---|---|---|
| Ex. 1 | MM | D-Leu | - | L-Arg | AcOH |
| Ex. 2 | EM | D-Leu | - | L-Arg | AcOH |
| Ex. 3 | MM | D-CHA | - | L-Arg | AcOH |
| Ex. 4 | MM | D-Nle | - | L-Lys | AcOH |
| Ex. 5 | MM | D-CHT | - | L-Arg | AcOH |
| Ex. 6 | MM | D-Nva | - | L-Arg | AcOH |
| Ex. 7 | MM | D-CHG | - | L-Arg | HCl |
| Ex. 8 | MM | D-Phe | - | L-Arg | HCl |
| Ex 9 | MM | D-Ala | - | L-Arg | HCl |
| Ex 10 | MM | D-Ile | - | L-Arg | AcOH |

**TABLEAU I (suite 2)**

| Produit | Q | $A_1$ | $A_2$ | $A_4$ | HA |
|---|---|---|---|---|---|
| Ex.11 | MM | D-Lys(Cbo) | - | L-Arg | AcOH |
| Ex.12 | MM | D-CHG | L-Asp(Py) | L-Lys | HCl |
| Ex 13 | MM | D-CHT | - | L-Arg | H-TFA |
| Ex 14 | MM | D-Nle | - | L-Arg | HCl |
| Ex 15 | EM | D-CHA | - | L-Arg | HCl. |
| Ex 16 | EM | D-CHT | - | L-Arg | HCl |
| Ex 17 | EM | D-Nva | - | L-Arg | AcOH |
| Ex 18 | EM | D-CHG | - | L-Arg | AcOH |
| Ex 19 | EM | D-Ala | - | L-Arg | AcOH |
| Ex 20 | EM | D-Ile | - | L-Arg | HCl |
| Ex 21 | EM | D-Nle | - | L-Arg | HCl |

**TABLEAU I (suite 3)**

| Produit | Q | $A_1$ | $A_2$ | $A_4$ | HA |
|---|---|---|---|---|---|
| Ex 22 | MM | D-Ile | - | L-Lys | AcOH |
| Ex 23 | MM | D-CHG | - | L-Lys | AcOH |
| Ex 24 | MM | D-Nva | - | L-Lys | HCl |
| Ex 25 | MM | D-Lys(Boc) | - | L-Arg | AcOH |
| Ex 26 | MM | D-Ala | - | L-Arg | HCl |
| Ex 27 | MM | D-Nle | L-Glu(Py) | L-Arg | HCl |
| Ex 28 | MM | D-Nle | L-Glu(OMe) | L-Arg | AcOH |
| Ex 29 | MM | D-Nle | L-Asp(Py) | L-Arg | AcOH |
| Ex 30 | MM | D-Nle | L-Glu | L-Arg | HCl |
| Ex 31 | MM | D-Leu | L-Glu(OMe) | L-Arg | AcOH |
| Ex 32 | MM | D-Ile | L-Glu | L-Arg | AcOH |
| Ex 33 | MM | D-Ile | L-Glu(OMe) | L-Arg | AcOH |
| Ex 34 | MM | D-Nva | L-Glu(Mor) | L-Arg | AcOH |

**TABLEAU I (suite 4)**

| Produit | Q | $A_1$ | $A_2$ | $A_4$ | HA |
|---|---|---|---|---|---|
| Ex 35 | MM | D-CHT | L-Asp(OMe) | L-Arg | AcOH |
| Ex 36 | MM | D-Leu | L-Asp | L-Lys | AcOH |
| Ex 37 | MM | D-Lys(Cbo) | L-Glu(Pi) | L-Arg | HCl |
| Ex 38 | MM | D-Lys(Cbo) | L-Asp(Pi) | L-Arg | HCl |
| Ex 39 | MM | D-CHA | L-Glu | L-Arg | AcOH |
| Ex 40 | MM | D-CHG | L-Glu(Py) | L-Arg | AcOH |
| Ex 41 | MM | D-Ala | L-Glu(OtBu) | L-Arg | AcOH |
| Ex 42 | MM | D-Ala | L-Asp | L-Arg | AcOH |
| Ex 43 | MM | D-Leu | L-Glu(Py) | L-Arg | HCl |
| Ex 44 | MM | D-Leu | L-Asp(OMe) | L-Arg | HCl |
| Ex 45 | MM | D-Ile | L-Asp(Py) | L-Arg | AcOH |
| Ex 46 | MM | D-Phe | L-Glu | L-Arg | AcOH |
| Ex 47 | MM | D-Phe | L-Glu(OMe) | L-Arg | AcOH |

**TABLEAU I (fin)**

| Produit | Q | $A_1$ | $A_2$ | $A_4$ | HA |
|---|---|---|---|---|---|
| Ex 48 | MM | D-Phe | L-Asp | L-Arg | HCl |
| Ex 49 | MM | D-Phe | L-Asp(Pi) | L-Arg | HCl |
| Ex 50 | MM | D-Nle | L-Glu(Mor) | L-Lys | AcOH |
| Ex 51 | MM | D-Nle | L-Asp | L-Arg | AcOH |
| Ex 52 | MM | D-Lys(Cbo) | L-Glu | L-Arg | AcOH |
| Ex 53 | MM | D-Lys(Cbo) | L-Glu(OMe) | L-Arg | AcOH |
| Ex 54 | MM | D-Lys(Cbo) | L-Glu(Py) | L-Arg | AcOH |
| Ex 55 | MM | D-Lys(Cbo) | L-Asp | L-Arg | AcOH |
| Ex 56 | MM | D-Lys(Cbo) | L-Asp(Py) | L-Arg | AcOH |
| Ex 57 | MM | D-Nva | L-Glu(OMe) | L-Arg | AcOH |
| Ex 58 | MM | D-Nva | L-Asp | L-Arg | AcOH |
| Ex 59 | MM | D-CHG | L-Asp(Py) | L-Arg | AcOH |
| Ex 60 | EM | D-CHG | L-Asp | L-Arg | AcOH |
| CP1 | $CH_3SO_2$ | D-Leu | - | L-Arg | AcOH |

**TABLEAU II**

$R_2-O(CH_2CH_2O)_n-CO-A_1-A_2-Gly-A_4-pNA, HA$

| Produit | $R_2$ | n | $A_1$ | $A_2$ | $A_4$ | HA |
|---|---|---|---|---|---|---|
| Ex 61 | Me | 1 | D-Leu | - | L-Arg | AcOH |
| Ex 62 | Me | 2 | D-Leu | - | L-Arg | AcOH |
| Ex 63 | Me | 3 | D-Leu | - | L-Arg | AcOH |
| Ex 64 | Me | 7 | D-Leu | - | L-Arg | AcOH |
| Ex 65 | Me | 12 | D-Leu | - | L-Arg | AcOH |
| Ex 66 | Me | 16 | D-Leu | - | L-Arg | AcOH |
| Ex 67 | Me | 150 | D-Leu | - | L-Arg | AcOH |
| Ex 68 | Me | 161 | D-Leu | - | L-Arg | AcOH |
| Ex 69 | Me | 113 | D-Leu | - | L-Arg | AcOH |
| Ex 70 | Me | 45 | D-Leu | - | L-Arg | AcOH |

**TABLEAU II (suite 1)**

$R_2-O(CH_2CH_2O)_n-CO-A_1-A_2-Gly-A_4-pNA, HA$

| Produit | $R_2$ | n | $A_1$ | $A_2$ | $A_4$ | HA |
|---|---|---|---|---|---|---|
| Ex 71 | Me | 1 | D-Nle | - | L-Arg | AcOH |
| Ex 72 | Me | 2 | D-Nle | - | L-Arg | AcOH |
| Ex 73 | Me | 3 | D-Nle | - | L-Arg | AcOH |
| Ex 74 | Me | 45 | D-Nle | - | L-Arg | AcOH |
| Ex 75 | Me | 2 | D-CHA | - | L-Arg | AcOH |
| Ex 76 | Me | 7 | D-CHA | - | L-Arg | AcOH |
| Ex 77 | Me | 113 | D-CHA | - | L-Arg | AcOH |
| Ex 78 | Me | 2 | D-CHT | - | L-Arg | AcOH |
| Ex 79 | Me | 3 | D-CHT | - | L-Arg | AcOH |
| Ex 80 | Me | 7 | D-Ala | - | L-Arg | AcOH |

**TABLEAU II (suite 2)**

$R_2-O(CH_2CH_2O)_n-CO-A_1-A_2-Gly-A_4-pNA, HA$

| Produit | $R_2$ | n | $A_1$ | $A_2$ | $A_4$ | HA |
|---|---|---|---|---|---|---|
| Ex 81 | Me | 1 | D-Leu | L-Glu(Py) | L-Arg | HCl |
| Ex 82 | Me | 7 | D-Leu | L-Glu(OMe) | L-Arg | HCl |
| Ex 83 | Me | 16 | D-Leu | L-Glu(Pi) | L-Arg | HCl |
| Ex 84 | Et | 2 | D-Nle | L-Asp(Py) | L-Arg | HCl |
| Ex 85 | Et | 7 | D-Nle | L-Asp(OMe) | L-Arg | HCl |
| Ex 86 | Et | 113 | D-Nle | L-Asp(Py) | L-Lys | HCl |
| Ex 87 | Et | 2 | D-CHA | L-Glu(Py) | L-Lys | HCl |
| Ex 88 | Et | 7 | D-CHA | L-Glu(OMe) | L-Lys | HCl |
| Ex 89 | Et | 2 | D-CHA | L-Glu(Pi) | L-Lys | HCl |
| Ex 90 | Et | 113 | D-CHA | L-Glu | L-Lys | HCl |

**TABLEAU II (suite 3)**

$R_2-O(CH_2CH_2O)_n-CO-A_1-A_2-Gly-A_4-pNA, HA$

| Produit | $R_2$ | n | $A_1$ | $A_2$ | $A_4$ | HA |
|---|---|---|---|---|---|---|
| Ex 91 | Et | 2 | D-Lys(Cbo) | L-Glu | L-Arg | HCl |
| Ex 92 | Et | 7 | D-Lys(Cbo) | L-Glu | L-Arg | HCl |
| Ex 93 | Et | 2 | D-Nva | L-Glu | L-Arg | HCl |
| Ex 94 | Et | 2 | D-Nva | L-Asp | L-Arg | HCl |
| Ex 95 | Et | 7 | D-Phe | L-Glu | L-Arg | HCl |
| Ex 96 | Et | 7 | D-Phe | L-Asp | L-Arg | HCl |
| Ex 97 | Et | 7 | D-Ile | L-Glu | L-Arg | HCl |
| Ex 98 | Et | 7 | D-CHG | L-Asp | L-Arg | HCl |
| Ex 99 | Bu | 1 | D-Leu | - | L-Arg | HCl |
| Ex 100 | Bu | 7 | D-Leu | - | L-Arg | HCl |

**TABLEAU II (fin)**

$R_2-O(CH_2CH_2O)_n-CO-A_1-A_2-Gly-A_4-pNA, HA$

| Produit | $R_2$ | n | $A_1$ | $A_2$ | $A_4$ | HA |
|---|---|---|---|---|---|---|
| Ex 101 | Bu | 7 | D-Nle | - | L-Arg | AcOH |
| Ex 102 | Bu | 12 | D-Nle | - | L-Arg | AcOH |
| Ex 103 | n-hexyle | 1 | D-Leu | - | L-Arg | AcOH |
| Ex 104 | n-hexyle | 7 | D-Leu | - | L-Arg | AcOH |
| Ex 105 | n-hexyle | 1 | D-CHA | - | L-Arg | AcOH |
| Ex 106 | n-hexyle | 7 | D-CHA | - | L-Arg | AcOH |

Les composés tri- et tétrapeptidiques suivant l'invention peuvent être préparés selon une méthode connue en soi par application de mécanismes réactionnels classiques. Le procédé de préparation que l'on préconise ici est caractérisé en ce que l'on fait réagir un peptide de formule

$H-A_1-A_2-Gly-A_4-R$ (IV)

où $A_1$, $A_2$, $A_4$ et R sont définis comme indiqué ci-dessus, avec une substance choisie parmi l'ensemble constitué par

a) les dérivés oxymalonyle de formule

$R_1-O-CO-CH_2-CO-T$ (V)

où $R_1$ est défini comme indiqué ci-dessus, et, T représente OH, F, Cl ou Br, et

b) les dérivés de PEG de formule

$R_2$-O($CH_2CH_2O$)$_n$-CO-Hal (VI)

où $R_2$ et n sont définis comme ci-dessus, et Hal représente F, Cl ou Br.

Les réactions IV + V = I et IV + VI = I sont chacune effectuées dans un solvant inerte, tel que DMF ou THF, en présence d'un excès d'une base agissant comme co-solvant et comme accepteur de protons telle que $Et_3N$, DIEA ou toute autre amine tertiaire convenable.

De façon pratique, pour la mise en oeuvre de la réaction IV + V = I, on préconise deux techniques différentes en fonction de la nature du groupe T. Quand T est F, Cl ou Br, on utilisera un rapport molaire IV/V inférieur ou égal à 1/2. Quand T est OH, on opèrera en outre en présence d'un agent couplant tel que Bop ou HOBT (DCCI étant ajouté le cas échéant à l'agent couplant HOBT lorsque celui-ci est utilisé) à une température d'environ 0 °C pendant au moins 1 heure puis à RT pendant au moins 24 heures, avec un rapport molaire IV/V compris dans la gamme allant de 1/1 à 1/1,5.

De plus, quand dans ladite réaction IV + V = I, T est F, Cl ou Br, on utilisera un rapport molaire IV/$Et_3N$ compris entre 1,7/1 à 3,6/1 et mieux de 2/1 à 3,1/1.

De façon pratique, la réaction IV + VI = I sera effectuée avec un rapport molaire IV/VI inférieur ou égal à 1 et de préférence inférieur ou égal à 0,8.

Pour la mise en oeuvre de la réaction IV + VI = I, on préfère faire appel à un dérivé de formule VI dans lequel Hal est Cl. Un tel dérivé chloré, utilisé comme matière première pour ladite réaction IV + VI = I, peut être synthétisé selon le schéma réactionnel

$$R_2\text{-O}(CH_2CH_2O)_n\text{-H}$$

$$\downarrow COCl_2$$

$$R_2\text{-O}(CH_2CH_2O)_n\text{-CO-Cl}$$

Les composés de formule V sont préparés à partir de l'acide malonique, d'une part, et les composés de formule IV sont obtenus selon une méthode classique de la synthèse peptidique comme décrit notamment dans le document EP-A-0 280 610 précité, d'autre part.

Les composés selon l'invention sont utiles dans la détermination du Facteur Xa. On préconise donc également un nécessaire, kit ou trousse de dosage destiné à la détermination du Facteur Xa, qui renferme au moins un composé peptidique choisi parmi l'ensemble constitué par les composés de formule I et leurs sels d'addition, et le cas échéant, un échantillon étalon de Facteur Xa et de milieux tamponnés de dilution.

On préconise également un procédé de détermination du Facteur Xa, ledit procédé étant caractérisé en ce que l'on met en contact dans un milieu biologique aqueux une quantité donnée d'un composé de formule I ou de l'un de ses sels d'addition et un échantillon à tester (éventuellement dilué) susceptible de contenir du Facteur Xa.

D'autres avantages et caractéristiques de l'invention seront mieux compris à la lecture qui va suivre de la description d'exemples de préparation et de résultats d'essais comparatifs. L'ensemble de ces éléments n'est nullement limitatif mais est donné à titre d'illustration.

**PREPARATION I**

Obtention de MM-D-Leu-Gly-L-Arg-pNA, AcOH

(Exemple 1)

a) Z-L-Arg-pNA, HCl

On dissout 344 g (1 mole) de Z-L-Arg-OH, HCl dans du HMPT anhydre (fraichement distillé et séché sur tamis moléculaire) à RT, puis on ajoute 139 ml (1 mode) de $Et_3N$ à RT sous agitation. A la solution résultante, on ajoute 328 g (2 moles) de p-nitrophénylisocyanate. Le milieu réactionnel résultant est maintenu sous agitation pendant 24 h à RT, puis est évaporé sous vide et repris avec une quantité minimale de AcOH avant d'être dilué avec AcOEt. La solution résultante est ensuite extraite successivement trois fois avec de petites quantités de $NaHCO_3$ 0,5 M, trois fois avec une solution de $KHSO_4$ à 50 g/l puis plusieurs fois avec $H_2O$ à demi-saturée en NaCl. La phase organique est ensuite séchée sur sulfate de

sodium anhydre. Après filtration (élimination de $Na_2SO_4$), on évapore le solvant et recristallise de résidu d'évaporation dans le mélange AcOEt/MeOMe (3/7) v/v. On obtient 350 g du produit attendu sous la forme d'une poudre de couleur blanche. F = 128-130 °C.

Analyse (CCM sur gel de silice) :

Rf = 0,5 dans AcOEt/pyridine/AcOH/$H_2O$ (20/4,5/3/1) v/v;

Rf = 0,69 dans $CHCl_3$/MeOH/AcOH (5/3/1) v/v.

b) H-L-Arg-pNA, 2HBr

Dans un appareillage en verre et téflon, on charge 100 g (0,215 moles) de Z-L-Arg-pNA, HCl. On ajoute, sous atmosphère inerte (courant d'azote), successivement 800 ml de AcOH glacial, 200 ml d'anisole, 1000 ml de HBr en solution dans AcOH glacial. On laisse réagir pendant 1 h à RT sous atmosphère d'azote. Après ce laps de temps, le mélange réactionnel, qui est devenu homogène au cours de la déprotection, est précipité dans 20 l d'éther (MeOMe ou EtOEt). Après décantation, le surnageant est écarté et le précipité est lavé plusieurs fois à l'éther. On recueille le précipité par filtration, le sèche sous vide sur KOH pendant 24 h. On obtient 94,2 g (rendement : 96 %) du produit attendu .

Analyse (CCM sur gel de silice) :

Rf = 0,04 dans AcOEt/pyridine/AcOH/$H_2O$ (20/4,5/3/1,5) v/v

Rf = 0,38 dans BuOH/AcOH/$H_2O$ (3/1/1) v/v.

c) Boc-Gly-L-Arg-pNA, HBr

On dissout 1 g (2,19 mmoles) de H-L-Arg-pNA, 2HBr dans 10 ml de DMF puis on ajoute 0,854 ml (6,57 mmoles) de DIEA. Dans un autre récipient, on neutralise 384 mg (2,19 mmoles) de Boc-Gly-OH dissous dans 5 ml de DMF au moyen d'une quantité de 0,285 ml de DIEA. On mélange les deux solutions ainsi obtenues et au milieu résultant on ajoute 970 mg de Bop tout en le maintenant à RT; on maintient également de pH à une valeur comprise entre 7 et 8 par ajout de petites portions de DIEA au cours de la réaction. Au bout d'une heure, la réaction est complètement terminée et on évapore à sec sous vide le milieu réactionnel; on reprend le résidu d'évaporation par un mélange AcOEt/MeOH et extrait au moyen d'une solution aqueuse de $NaHCO_3$ 0,5 M. On sèche la phase organique sur sulfate de sodium, la concentre sous vide puis précipite dans d'éther (MeOMe ou EtOEt). On obtient 874 mg (rendement : 75 %) de produit attendu.

Analyse (CCM sur gel de silice) :

Rf = 0,53 dans $CHCl_3$/MeOH/AcOH (10/3/1) v/v.

d) H-Gly-L-Arg-pNA, 2H-TFA

On charge dans un réacteur 874 mg (1,64 mmoles) de Boc-Gly-L-Arg-pNA, HBr puis ajoute successivement 6,6 ml de $CH_2Cl_2$ et 6,6 ml de H-TFA. Après 0,25 h de réaction à RT, on précipite directement le mélange réactionnel dans l'éther. Il se forme un précipité blanc floconneux que l'on filtre et sèche. On obtient 910 mg (rendement : 96 %) de produit attendu.

Analyse (CCM sur gel de silice) :

Rf = 0,09 dans $CHCl_3$/MeOH/AcOH (5/3/1) v/v.

e) Boc-D-Leu-Gly-L-Arg-pNA, H-TFA

En suivant les modalités opératoires de la préparation Ic, on fait réagir un mélange réactionnel comprenant (i) 910 mg (1,57 mmoles) de H-Gly-L-Arg-pNA, 2H-TFA, (ii) 363 mg (1,57 mmoles) de Boc-D-Leu-OH, (iii) 1,2 ml de DIEA et (iv) 695 mg (1,57 mmoles) de Bop dans le DMF, et on maintient le mélange réactionnel à RT sous agitation pendant 2 h. Après évaporation à sec sous vide, le résidu d'évaporation est chromatographié sur gel de silice avec comme éluant un gradient de $CHCl_3$/MeOH/AcOH de (20/3/1) à (10/3/1) v/v. On réunit les fractions homogènes recueillies et évapore l'éluant. Par lyophilisation, on obtient 785 mg (rendement : 80 %) du produit pur attendu.

Analyse (CCM sur gel de silice) :

Rf = 0,33 dans $CHCl_3$/MeOH/AcOH (10/3/1) v/v.

f) H-D-Leu-Gly-L-Arg-pNA, 2H-TFA

On fait réagir 785 mg (1,25 mmoles) de Boc-D-Leu-Gly-L-Arg-pNA, H-TFA obtenu selon la préparation le avec 5 ml de $CH_2Cl_2$ et 5 ml de H-TFA à RT. Au bout de 0,25 h, le mélange réactionnel est directement précipité dans l'éther. On obtient un précipité blanc que l'on filtre, lave à l'éther et sèche. On recueille 779 mg (rendement : 90 %) du produit attendu.

Analyse (CCM sur gel de silice) :
Rf = 0,2 dans $CHCl_3$/MeOH/AcOH (5/3/1) v/v.

g) MM-D-Leu-Gly-L-Arg-pNA, AcOH

Dans un récipient de réaction, on charge 7,79 mg (1,12 mmoles) de H-D-Leu-Gly-L-Arg-pNA, 2H-TFA puis ajoute 15 ml de DMF et 0,517 ml (3,705 mmoles) de $Et_3N$. On refroidit le mélange résultant à -30 °C sous agitation puis introduit goutte-à-goutte (toujours sous agitation et à -30 °C) 0,265 ml de chlorure de méthyloxymalonyle (MeO-CO-$CH_2$-CO-Cl). Le mélange résultant est maintenu à -30 °C pendant 0,5 h puis on le laisse se réchauffer jusqu'à RT. Après 2 h de réaction, le mélange réactionnel est filtré pour éliminer le sel de triéthylammonium qui s'est formé et le filtrat est évaporé sous vide. Le résidu d'évaporation est précipité au moyen d'éther (EtOEt), filtré puis chromatographié sur résine échangeuse d'ions (AMBERLITE[R] IRA 401 S) préalablement acétylée, avec comme éluant le mélange MeOH/$H_2O$ (3/2) v/v. Les fractions homogènes sont réunies et évaporées. Par lyophilisation du résidu d'évaporation résultant, on obtient 566 mg (rendement : 81 %) du produit attendu.

Analyse :
CCM sur gel de silice :
Rf = 0,71 dans $CHCl_3$/MeOH/AcOH (10/3/1) v/v;
HPLC [sur colonne d'HYPERSIL[R] C 18 (granulométrie : 3 µm) fourni par la société dite MERCK] :
TR = 10 minutes [avec solution isocrotique contenant eau (72,4 %) acétonitrile (27,5 %) et AcOH (0,2 % p/p)].

**PREPARATION II**

Obtention de MM-D-Leu-L-Arg-pNA, AcOH

(Exemple 1)

On procède comme indiqué dans la préparation I mais en remplaçant l'étape Ig par l'étape suivante.

Dans un récipient de réaction, on charge 1391 mg (2 mmoles) de H-D-Leu-Gly-L-Arg-pNA, 2H-TFA puis introduit 30 ml de DMF et 0,7 ml (5 mmoles) de $Et_3N$. On ajoute à la température ambiante 973,5 mg de Bop puis ajoute au mélange résultant une solution de 259 mg d'acide méthyloxymalonyle (MeO-CO-$CH_2$-CO-OH) dans 10 ml de DMF et 0,7 ml de $Et_3N$. Pendant l'évolution de la réaction, on maintient le pH entre 7 et 8 pendant le couplage par ajout successif de petites quantités de $Et_3N$; après 3 h de réaction sous agitation, le couplage est terminé. On extrait le milieu réactionnel au moyen d'un système solvant AcOEt/$KHSO_4$ (à 5 % p/v) puis AcOEt/$NaHCO_3$ 0,5 M. On lave ensuite la phase AcOEt avec de l'eau à demisaturée en NaCl. La phase AcOEt est évaporée sous vide puis le résidu d'évaporation est chromatographié sur colonne de silice (granulométrie : 40-60 micromètres) au moyen du système éluant $CHCl_3$/MeOH/AcOH (8/3/1) v/v. Les fractions homogènes sont réunies et évaporées. Par lyophilisation du résidu d'évaporation résultant, on obtient 812 mg (rendement : 65 %) du produit attendu.

Analyse :
CCM sur gel de silice :
Rf = 0,71 dans $CHCl_3$/MeOH/AcOH (10/3/1) v/v;
HPLC [comme indiqué plus haut au stade Ig] :
TR = 10 minutes.

**PREPARATION III**

Obtention de Me-$OCH_2CH_2O$-CO-D-Leu-Gly-L-Arg-pNA, AcOH

(Exemple 61)

a) Et-$OCH_2CH_2O$-CO-Cl

Dans un réacteur à une température de -30 ° C, on condense le phosgène puis ajoute goutte-à-goutte (toujours à -30 ° C), au moyen d'une ampoule de coulée, 10 ml de l'éther monométhylique d'éthylèneglycol préalablement dissous dans du THF, de façon à ce que le phosgène soit toujours dans un large excès par rapport à l'alcool. On maintient le milieu réactionnel sous agitation à -30 ° C pendant 2 h puis on le laisse se réchauffer jusqu'à RT. L'excès de phosgène est éliminé par un courant d'azote opérant pendant plusieurs heures afin d'éliminer toute trace dudit phosgène. Le mélange réactionnel est ensuite évaporé sous vide et le liquide obtenu est utilisé tel quel dans l'étape suivante de couplage. On obtient 1320 mg (rendement : 95 %) du produit attendu.

Analyse (spectre IR) :

- disparition de la bande alcool à 3300 $cm^{-1}$;
- apparition des bandes chloroformiates à 1776, 1152 et 690 $cm^{-1}$.

Remarque : Le procédé de l'étape IIIa est directement utilisable pour obtenir les autres chloroformiates de formule $R_2$-$O(CH_2CH_2O)_n$-CO-Cl

b) $MeOCH_2CH_2O$-CO-D-Leu-Gly-L-Arg-pNA, AcOH

Dans un récipient de réaction, on charge 1391 mg (2 mmoles) de H-D-Leu-Gly-L-Arg-pNA, 2H-TFA (obtenu comme indiqué dans la préparation If) puis ajoute 30 ml de DMF et 0,624 ml de $Et_3N$. Le mélange résultant est refroidi à 0 ° C au moyen d'un bain glace. On ajoute audit mélange ainsi refroidi 0,304 g (2,2 mmoles) de chloroformiate $MeOCH_2CH_2O$-CO-Cl. On maintient le milieu réactionnel sous agitation pendant 1 h, on le laisse se réchauffer jusqu'à RT puis poursuit la réaction pendant 1 h à RT. Le mélange réactionnel est ensuite filtré, évaporé sous vide et le résidu d'évaporation est chromatographié sur une résine échangeuse d'ions (AMBERLITE[R] IRA 400 S) préalablement acétylée, avec comme éluant le mélange MeOH/$H_2O$ (3/2) v/v. Les fractions homogènes sont réunies et évaporées. Par lyophilisation du résidu d'évaporation résultant, on obtient 814 mg (rendement : 65 %) du produit attendu.

Analyse :

CCM sur gel de silice :

Rf = 0,65 dans $CHCl_3$/MeOH/AcOH (10/3/1) v/v.

**PREPARATION IV**

Obtention de Me-$O(CH_2CH_2O)_7$-CO-D-Leu-Gly-L-Arg-pNA, AcOH

(Exemple 64)

On procède comme indiqué à la préparation IIIb en remplaçant le Me-$OCH_2CH_2O$-CO-Cl par le MeO-$(CH_2CH_2O)_7$-COCl pour obtenir 1,038 g (rendement : 61 %) du produit attendu.

Analyse (CCM sur gel de silice) :

Rf = 0,61 dans $CHCl_3$/MeOH/AcOH (10/3/1) v/v;

Rf = 0,14 dans $CHCl_3$/MeOH/AcOH (20/3/1) v/v.

**PREPARATION V**

Obtention de Me-$O(CH_2CH_2O)_2$-CO-D-Leu-Gly-L-Arg-pNA, AcOH

(Exemple 62)

On procède comme indiqué à la préparation IIIb en remplaçant le Me-$OCH_2CH_2O$-CO-Cl par le MeO-$(CH_2CH_2O)_2$-COCl pour obtenir 851 mg (rendement : 68 %) du produit attendu.

Analyse (CCM sur gel de silice) :
Rf = 0,45 dans $CHCl_3$/MeOH/AcOH (15/3/1) v/v.

**ESSAIS COMPARATIFS**

L'activité des peptides suivant l'invention vis-à-vis du Facteur Xa a été déterminée selon une des méthodes classiques, telle que décrite dans EP-A-0 280 160 (voir page 14), en appréciant la vitesse d'hydrolyse par la variation de la densité optique dans le temps (ΔOD/min). Les résultats qui ont été obtenus à des doses équimolaires ont été consignés dans le tableau III ci-après où par commodité, l'activité du produit de référence CP 1 est précisée comme étant égale à 100 % .

Les résultats comparatifs du tableau III mettent en évidence que les peptides suivant l'invention sont au moins aussi actifs que le produit de référence CP 1.

TABLEAU III

| ACTIVITE VIS-A-VIS DU FACTEUR Xa | |
|---|---|
| Produit | Activité |
| Ex 1 | 115 % |
| Ex 2 | 132 % |
| Ex 14 | 140 % |
| Ex 33 | 105 % |
| Ex 61 | 145 % |
| Ex 62 | 105 % |
| Ex 63 | 110 % |
| Ex 64 | 103 % |
| Ex 73 | 121 % |
| Ex 101 | 124 % |
| CP 1 | 100 % |

**Revendications**

**1.** Composé peptidique appartenant à la famille des substrats tri- et tétrapeptidiques, caractérisé en ce qu'il est choisi parmi l'ensemble constitué par

(i) les tri- et tétrapeptides répondant à la formule

$Q\text{-}A_1\text{-}A_2\text{-Gly-}A_4\text{-}R$ (I)

dans laquelle

Q est un groupe bloquant l'extrémité N-terminale de la chaîne peptidique et qui est choisi parmi l'ensemble constitué par

(a) un reste oxymalonyle de formule $R_1\text{-O-CO-CH}_2\text{-CO}$, où $R_1$ est
- un groupe alkyle en $C_1$-$C_4$,
- un groupe phényle,
- un groupe phényle substitué par un ou plusieurs groupes Me, MeO, Cl, Br, F, $CF_3$,
- un groupe cycloalkyle en $C_3$-$C_6$,
- un groupe benzyle,
- un groupe benzyle substitué par un ou plusieurs groupes Me, MeO, Cl, Br, F, $CF_3$, ou
- un groupe cycloalkylméthyle où le fragment cycloalkyle est en $C_3$-$C_6$ ; et

(b) un reste de polyéthylèneglycol de formule $R_2\text{-O}(CH_2CH_2O)_n\text{-CO}$, où $R_2$ est un groupe alkyle en $C_1$-$C_6$, phényle ou benzyle et n est un nombre entier ayant pour valeur 1 à 170,

$A_1$ est choisi parmi l'ensemble constitué par les groupes Leu, Ile, Nle, Nva, CHA, CHG, CHT, Phe, Ala et Lys où la fonction basique latérale du reste Lys est bloquée par un groupe convenable éliminant substantiellement le caractère basique de ladite fonction latérale,

$A_2$ est une simple liaison, Asp ou Glu, le groupe acide carboxylique latéral de Asp et Glu étant susceptible d'être estérifié ou amidifié,

$A_4$ est choisi parmi d'ensemble constitué par des groupes Arg et Lys,
R est un moyen de marquage clivable ; et
(ii) leurs sels d'addition.

**2.** Composé peptidique suivant la revendication 1, caractérisé en ce que, dans de reste $R_1$-O-CO-$CH_2$-CO, $R_1$ est choisi parmi l'ensemble constitué par les groupes alkyle en $C_1$-$C_4$, phényle, tolyle, xylyle, 2-, 3- ou 4-méthoxyphényle, 2,4- 2,6- 3,4- ou 3,5-diméthoxyphényle, 4-chlorophényle, 4-fluorophényle, 3,4-, 3,5- ou 2,6-dichlorophényle, 3-trifluorométhylphényle, cyclopropyle, cyclopentyle, cyclohexyle, benzyle, 2-, 3- ou 4-méthylbenzyle, 2-, 3- ou 4-méthoxybenzyle, 2,4-, 2,6-, 3,4- ou 3,5-diméthylbenzyle, 2,4-, 2,6-, 3,4- ou 3,5-diméthoxybenzyle, 2-, 3- ou 4-chlorobenzyle, 2-, 3- ou 4-fluorobenzyle, 2,4-, 2,6-, 3,4- ou 3,5-dichlorobenzyle, 3-trifluorométhylbenzyle, cyclopropylméthyle, cyclopentylméthyle et cyclohexylméthyle.

**3.** Composé peptidique suivant la revendication 1, caractérisé en ce que, dans le reste $R_2$-O($CH_2CH_2O)_n$-CO, n est un nombre entier ayant pour valeur 1 à 161.

**4.** Composé peptidique suivant la revendication 1, caractérisé en ce que $A_1$ est choisi parmi l'ensemble constitué par les groupes D-Leu, D-Ile, D-Nle, D-Nva, D-CHA, D-CHG, D-CHT, D-Phe, D-Ala et D-Lys où la fonction basique latérale de D-Lys est bloquée par un groupe acide convenable.

**5.** Composé peptidique suivant la revendication 1, caractérisé en ce que $A_1$ est D-Leu, D-Nle ou D-CHA.

**6.** Composé peptidique suivant la revendication 1, caractérisé en ce que $A_2$ est une simple liaison, L-Glu, L-Asp, L-Glu(Mor), L-Glu(Py), L-Glu(Pi), L-Glu(OMe), L-Glu (OEt), L-Glu(OtBu), L-Asp(Mor), L-Asp(Py), L-Asp(Pi), L-Asp(OMe), L-Asp(OEt) ou L-Asp(OtBu).

**7.** Composé peptidique suivant la revendication 1, caractérisé en ce qu'il est choisi parmi l'ensemble constitué par

(i) les composés tri- ou tétrapeptidiques répondant à la formule

Q-$A_1$-$A_2$-Gly-$A_4$-pNA (III)

dans laquelle

- Q est MM, EM ou $R_2$-O($CH_2CH_2O)_n$-CO où $R_2$ est Me, Et, Pr, iPr, Bu, iBu, ou n-hexyle et où n est un nombre entier compris entre 1 et environ 161,
- $A_1$ est choisi parmi l'ensemble constitué par D-Leu, D-Ile, D-Nle, D-Nva, D-CHA, D-CHG, D-CHT, D-Phe, D-Ala, D-Lys(Cbo) et D-Lys(Boc), les groupes $A_1$ préférés étant D-Nle, D-Leu et D-CHA,
- $A_2$ est choisi parmi d'ensemble constitué par une simple liaison et les groupes L-Glu et L-Asp où le groupe OH du reste acide carboxylique latéral desdits L-Glu et L-Asp peut être remplacé par OMe, OEt, OtBu, morpholino, pipéridino ou pyrrolidino,
- $A_4$ est choisi parmi d'ensemble constitué par L-Arg et L-Lys; et,

(ii) leurs sels d'addition d'acide.

**8.** Composé peptidique suivant la revendication 1, caractérisé en ce qu'il est choisi parmi l'ensemble constitué par

(a) MM-D-Leu-Gly-L-Arg-pNA,
(b) EM-D-Leu-Gly-L-Arg-pNA,
(c) MeO($CH_2CH_2O$)-CO-D-Leu-Gly-L-Arg-pNA,
(d) MeO($CH_2CH_2O)_2$-CO-D-Leu-Gly-L-Arg-pNA,
(e) MeO($CH_2CH_2O)_3$-CO-D-Leu-Gly-L-Arg-pNA,
(f) MeO($CH_2CH_2O)_7$-CO-D-Leu-Gly-L-Arg-pNA,
(g) MM-D-Nle-Gly-L-Arg-pNA,
(h) MM-D-Nle-L-Glu(Py)-Gly-L-Arg-pNA,
(i) MM-D-CHA-Gly-L-Arg-pNA,
(j) MM-D-Ile-L-Glu(OMe)-Gly-L-Arg-pNA,
(k) EM-D-Nle-Gly-L-Arg-pNA, et
(l) leurs sels d'addition d'acide.

9. Procédé de préparation d'un composé tri- ou tétrapeptique de formule I et de ses sels d'addition selon la revendication 1, ledit procédé étant caractérisé en ce que l'on fait réagir un peptide de formule

$H\text{-}A_1\text{-}A_2\text{-Gly-}A_4\text{-R}$ (IV)

où $A_1$, $A_2$, $A_4$ et R sont définis comme indiqué ci-dessus, avec une substance choisie parmi l'ensemble constitué par

a) les dérivés oxymalonyle de formule

$R_1\text{-O-CO-}CH_2\text{-CO-T}$ (V)

où $R_1$ est défini comme indiqué ci-dessus, et, T représente OH, F, Cl ou Br, et

b) les dérivés de PEG de formule

$R_2\text{-O}(CH_2CH_2O)_n\text{-CO-Hal}$ (VI)

où $R_2$ et n sont définis comme ci-dessus, et Hal représente F, Cl ou Br.

10. Utilisation d'un composé de formule I ou de l'un de ses sels d'addition selon la revendication 1, dans la détermination du Facteur Xa.

11. Procédé de détermination du Facteur Xa, ledit procédé étant caractérisé en ce que d'on met en contact dans un milieu biologique aqueux une quantité donnée d'un composé de formule I ou de l'un de ses sels d'addition selon la revendication 1 et un échantillon à tester susceptible de contenir du Facteur Xa.

12. Nécessaire de dosage destiné à la détermination du Facteur Xa, caractérisé en ce qu'il renferme au moins un composé peptidique choisi parmi d'ensemble constitué par les composés de formule I et leurs sels d'addition selon la revendication 1, et de cas échéant, un échantillon étalon de Facteur Xa et de milieux tamponnés de dilution.

**Claims**

1. A peptide compound belonging to the family of the tri- and tetra-peptide substrates, which is selected from the group consisting of

(i) the tri- and tetra-peptides of the formula

$Q\text{-}A_1\text{-}A_2\text{-Gly-}A_4\text{-R}$ (I)

in which

Q is a group which blocks the N-terminal end of the peptide chain and which is selected from the group consisting of

(a) an oxymalonyl radical of the formula $R_1\text{-O-CO-}CH_2\text{-CO}$, in which $R_1$ is

a $C_1$-$C_4$ alkyl group,

a phenyl group,

a phenyl group substituted by one or more Me, MeO, Cl, Br, F or $CF_3$ groups,

a $C_3$-$C_6$ cycloalkyl group,

a benzyl group,

a benzyl group substituted by one or more Me, MeO, Cl, Br, F or $CF_3$ groups, or

a cycloalkylmethyl group in which the cycloalkyl fragment is $C_3$-$C_6$; and

(b) a polyethylene glycol residue of the formula $R_2\text{-O}(CH_2CH_2O)_n\text{-CO}$, in which $R_2$ is a $C_1$-$C_6$ alkyl, phenyl or benzyl group and n is an integer having a value of 1 to 170,

$A_1$ is selected from the group consisting of Leu, Ile, Nle, Nva, CHA, CHG, CHT, Phe, Ala and Lys groups, where the basic side-group of the Lys residue is blocked with a suitable group which substantially removes the basic character of said side-group,

$A_2$ is a single bond, Asp or Glu, the carboxylic acid side-group of Asp and Glu being capable of esterification or amidation,

$A_4$ is selected from the group consisting of Arg and Lys groups, and

R is a cleavable labeling means; and

(ii) their addition salts.

**2.** A peptide compound according to claim 1 wherein, in the radical $R_1$-O-CO-$CH_2$-CO, $R_1$ is selected from the group consisting of $C_1$-$C_4$ alkyl, phenyl, tolyl, xylyl, 2-, 3- or 4-methoxyphenyl, 2,4-, 2,6-, 3,4- or 3,5-dimethoxyphenyl, 4-chlorophenyl, 4-fluorophenyl, 3,4-, 3,5- or 2,6-dichlorophenyl, 3-trifluoromethylphenyl, cyclopropyl, cyclopentyl, cyclohexyl, benzyl, 2-, 3- or 4-methylbenzyl, 2-, 3- or 4-methoxybenzyl, 2,4-, 2,6-, 3,4- or 3,5-dimethylbenzyl, 2,4-, 2,6-, 3,4- or 3,5-dimethoxybenzyl, 2-, 3- or 4-chlorobenzyl, 2-, 3- or 4-fluorobenzyl, 2,4-, 2,6-, 3,4- or 3,5-dichlorobenzyl, 3-trifluoromethylbenzyl, cyclopropylmethyl, cyclopentylmethyl and cyclohexylmethyl groups.

**3.** A peptide compound according to claim 1 wherein, in the residue $R_2$-O($CH_2CH_2O$)$_n$-CO, n is an integer having a value of 1 to 161.

**4.** A peptide compound according to claim 1 wherein $A_1$ is selected from the group consisting of D-Leu, D-Ile, D-Nle, D-Nva, D-CHA, D-CHG, D-CHT, D-Phe, D-Ala and D-Lys groups, where the basic side-group of D-Lys is blocked with a suitable acid group.

**5.** A peptide compound according to claim 1 wherein $A_1$ is D-Leu, D-Nle or D-CHA.

**6.** A peptide compound according to claim 1 wherein $A_2$ is a single bond, L-Glu, L-Asp, L-Glu(Mor), L-Glu-(Py), L-Glu(Pi), L-Glu(OMe), L-Glu(OEt), L-Glu(OtBu), L-Asp(Mor), L-Asp(Py), L-Asp(Pi), L-Asp(OMe), L-Asp(OEt) or L-Asp(OtBu).

**7.** A peptide compound according to claim 1 which is selected from the group consisting of
(i) the tri- or tetra-peptide compounds of the formula

Q-$A_1$-$A_2$-Gly-$A_4$-pNA (III)

in which
- Q is MM, EM or $R_2$-O($CH_2CH_2O$)$_n$-CO, in which $R_2$ is Me, Et, Pr, iPr, Bu, iBu or n-hexyl and in which n is an integer of between 1 and about 161,
- $A_1$ is selected from the group consisting of D-Leu, D-Ile, D-Nle, D-Nva, D-CHA, D-CHG, D-CHT, D-Phe, D-Ala, D-Lys(Cbo) and D-Lys(Boc), the preferred groups $A_1$ being D-Nle, D-Leu and D-CHA,
- $A_2$ is selected from the group consisting of a single bond and L-Glu and L-Asp groups, in which the OH group of the carboxylic acid side-radical of said L-Glu and L-Asp can be replaced with OMe, OEt, OtBu, morpholino, piperidino or pyrrolidino, and
- $A_4$ is selected from the group consisting of L-Arg and L-Lys; and

(ii) their acid addition salts.

**8.** A peptide compound according to claim 1 which is selected from the group consisting of
(a) MM-D-Leu-Gly-L-Arg-pNA,
(b) EM-D-Leu-Gly-L-Arg-pNA,
(c) MeO($CH_2CH_2O$)-CO-D-Leu-Gly-L-Arg-pNA,
(d) MeO($CH_2CH_2O$)$_2$-CO-D-Leu-Gly-L-Arg-pNA,
(e) MeO($CH_2CH_2O$)$_3$-CO-D-Leu-Gly-L-Arg-pNA,
(f) MeO($CH_2CH_2O$)$_7$-CO-D-Leu-Gly-L-Arg-pNA,
(g) MM-D-Nle-Gly-L-Arg-pNA,
(h) MM-D-Nle-L-Glu(Py)-Gly-L-Arg-pNA,
(i) MM-D-CHA-Gly-L-Arg-pNA,
(j) MM-D-Ile-L-Glu(OMe)-Gly-L-Arg-pNA,
(k) EM-D-Nle-Gly-L-Arg-pNA and
(1) their acid addition salts.

**9.** A method of preparing a tri- or tetra-peptide compound of formula I and its addition salts according to claim 1, said method comprising reacting a peptide of the formula

H-$A_1$-$A_2$-Gly-$A_4$-R (IV)

in which $A_1$, $A_2$, $A_4$ and R are defined as indicated above, with a substance selected from the group consisting of

a) the oxymalonyl derivatives of the formula

$R_1$-O-CO-$CH_2$-CO-T (V)

in which $R_1$ is defined as indicated above and T is OH, F, Cl or Br, and

b) the PEG derivatives of the formula

$R_2$-O($CH_2CH_2O$)$_n$-CO-Hal (VI)

in which $R_2$ and n are as defined above and Hal is F, Cl or Br.

**10.** Use of a compound of formula I or one of its addition salts according to claim 1 in the determination of Factor Xa.

**11.** A method of determining Factor Xa, wherein a given amount of a compound of formula I or of one of its addition salts according to claim 1 is brought into contact, in an aqueous biological medium, with a test sample which may contain Factor Xa.

**12.** An assay kit for the determination of Factor Xa, which contains at least one peptide compound selected from the group consisting of the compounds of formula I and their addition salts according to claim 1 and, if appropriate, a standard sample of Factor Xa and of buffered dilution media.

## Patentansprüche

**1.** Peptidzusammensetzung aus der Familie der Drei- und Vierpeptidsubstrate,
**dadurch gekennzeichnet,** daß
sie aus

(i) den Tri- und Tetrapeptiden der nachfolgenden Formel

Q-$A_1$-$A_2$-Gly-$A_4$-R (I),

in der

Q eine Schutzgruppe am N-terminalen Ende der Peptidkette ist und aus

(a) einem Oxymalonylrest der Formel $R_1$-O-CO-$CH_2$-CO, in der $R_1$ eine $C_1$-$C_4$-Alkylgruppe,
eine Phenylgruppe,
eine mit einer oder mehreren der Gruppen Me, MeO, Cl, Br, F, $CF_3$ substituierte Phenylgruppe,
eine $C_3$-$C_6$-Cycloalkylgruppe,
eine Benzylgruppe,
eine mit einer oder mehreren der Gruppen Me, MeO, Cl, Br, F, $CF_3$ substutierte Benzylgruppe oder
eine Cycloalkylmethylgruppe mit einem $C_3$-$C_6$-Cycloalkylrest ist, und

(b) einem Polyethylenglykolrest der Formel $R_2$-O($CH_2CH_2O$)$_n$-CO, in der $R_2$ eine $C_1$-$C_6$-Alkylgruppe, Phenyl oder Benzyl und n eine Zahl von 1 bis 170 ist, ausgewählt ist,

$A_1$ aus Leu, Ile, Nle, Nva, CHA, CHG, CHT, Phe, Ala und Lys ausgewählt ist, in der die basische Seitenfunktion des Lysinrestes durch eine geeignete Gruppe geschützt ist, die im wesentlichen die basischen Eigenschaften der genannten Seitenfunktion eliminiert,

$A_2$ eine Einfachbindung, Asp oder Glu ist, wobei die seitliche saure Carboxylgruppe von Asp und Glu der Veresterung oder Amidierung zugänglich ist,

$A_4$ aus Arg und Lys ausgewählt ist,

R ein Rest mit einer spaltbaren Gruppe ist und

(ii) ihren Additionssazen ausgewählt ist.

**2.** Peptidzusammensetzung nach Anspruch 1,
**dadurch gekennzeichnet,** daß
in dem Rest $R_1$-O-CO-$CH_2$-CO $R_1$ aus $C_1$-$C_4$-Alkylgruppen, Phenyl, Tolyl, Xylyl, 2-, 3- oder 4-Methoxyphenyl, 2,4-, 2,6-, 3,4- oder 3,5-Dimethoxyphenyl, 4-Chlorphenyl, 4-Fluorphenyl, 3,4-, 3,5- oder 2,6-Dichlorphenyl, 3-Trifluormethylphenyl, Cyclopropyl, Cyclopentyl, Cyclyhexyl, Benzyl, 2-, 3- oder 4-Methylbenzyl, 2-, 3- oder 4-Methoxybenzyl, 2,4-, 2,6-, 3,4- oder 3,5-Dimethylbenzyl, 2,4-, 2,6-, 3,4- oder 3,5-Dimethoxybenzyl, 2-, 3- oder 4-Chlorbenzyl, 2-, 3- oder 4-Fluorbenzyl, 2,4-, 2,6-, 3,4- oder 3,5-Dichlorbenzyl, 3-Trifluormethylbenzyl, Cyclopropylmethyl, Cyclopentylmethyl und Cyclohexylmethyl ausgewählt ist.

**3.** Peptidzusammensetzung nach Anspruch 1,
**dadurch gekennzeichnet,** daß
in dem Rest $R_2$-O($CH_2CH_2O)_n$-CO n eine Zahl von 1 bis 160 ist.

**4.** Peptidzusammensetzung nach Anspruch 1,
**dadurch gekennzeichnet,** daß
$A_1$ aus D-Leu, D-Ile, D-Nle, D-Nva, D-CHA, D-CHG, D-CHT, D-Phe, D-Ala und D-Lys ausgewählt ist, in der die basische Seitenfunktion des D-Lys durch eine geeignete Säuregruppe geschützt ist.

**5.** Peptidzusammensetzung nach Anspruch 1,
**dadurch gekennzeichnet,** daß
$A_1$ D-Leu, D-Nle oder D-CHA ist.

**6.** Peptidzusammensetzung nach Anspruch 1,
**dadurch gekennzeichnet,** daß
$A_2$ eine Einfachbindung, L-Glu, L-Asp, L-Glu(Mor), L-Glu(Py), L-Glu(Pi), L-Glu(OMe), L-Glu(OEt), L-Glu-(OtBu), L-Asp(Mor), L-Asp(Py), L-Asp(Pi), L-Asp(OMe), L-Asp(OEt) oder L-Asp(OtBu) ist.

**7.** Peptidzusammensetzung nach Anspruch 1,
**dadurch gekennzeichnet,** daß
sie aus
(i) Tri- oder Tetrapeptidzusammensetzungen der Formel

Q-$A_1$-$A_2$-Gly-$A_4$-pNA (III),

in der
- Q MM, EM oder $R_2$-O($CH_2CH_2O)_n$-CO ist, in der $R_2$ Me, Et, Pr, iPr, Bu, iBu oder n-Hexyl ist und n eine Zahl zwischen 1 und etwa 161 ist,
- $A_1$ aus D-Leu, D-Ile, D-Nle, D-Nva, D-CHA, D-CHG, D-CHT, D-Phe, D-Ala, D-Lys(Cbo) und D-Lys(Box), vorzugsweise D-Nle, D-Leu und D-CHA, ausgewählt ist,
- $A_2$ aus einer Einfachbindung und der L-Glu- und L-Asp-Gruppe ausgewählt ist, in der die OH-Gruppe des seitlichen carboxylsauren Restes der L-Glu und L-Asp durch OMe, OEt, OtBu, Morpholino, Piperidino oder Pyrrolidino ersetzt werden können,
- $A_4$ aus L-Arg und L-Lys ausgewählt ist und

(ii) ihren sauren Additionssalzen ausgewählt ist.

**8.** Peptidzusammensetzung nach Anspruch 1,
**dadurch gekennzeichnet,** daß
sie aus den folgenden Bestandteilen
(a) MM-D-Leu-Gly-L-Arg-pNA,
(b) EM-D-Leu-Gly-L-Arg-pNA,
(c) MeO($CH_2CH_2O$)-CO-D-Leu-Gly-L-Arg-pNA,
(d) MeO($CH_2CH_2O)_2$-CO-D-Leu-Gly-L-Arg-pNA,
(e) MeO($CH_2CH_2O)_3$-CO-D-Leu-Gly-L-Arg-pNA,
(f) MeO($CH_2CH_2O)_7$-CO-D-Leu-Gly-L-Arg-pNA,
(g) MM-D-Nle-Gly-L-Arg-pNA,
(h) MM-D-Nle-L-Glu(Py)-Gly-L-Arg-pNa,
(i) MM-D-CHA-Gly-L-Arg-pNA,
(j) MM-D-Ile-L-Glu(Ome)-Gly-L-Arg-pNA,
(k) EM-D-Nle-Gly-L-Arg-pNA und

(l) ihren sauren Additionssalzen
ausgewählt ist.

**9.** Verfahren zur Herstellung einer Tri- oder Tetrapeptidzusammensetzung der Formel I und ihrer Additionssalze nach Anspruch 1,
**gekennzeichnet durch**
Umsetzung eines Peptids der Formel

$H\text{-}A_1\text{-}A_2\text{-}Gly\text{-}A_4\text{-}R$ (IV),

in der $A_1$, $A_2$, $A_4$ und R wie vorstehend definiert sind, mit einer aus
a) Oxymalonylderivaten der Formel

$R_1\text{-}O\text{-}CO\text{-}CH_2\text{-}CO\text{-}T$ (V),

in der $R_1$ wie vorstehend definiert ist und T OH, F, Cl oder Br sind und
b) den PEG-Derivaten der Formel

$R_2\text{-}O(CH_2CH_2O)_n\text{-}CO\text{-}Hal$ (VI),

in der $R_2$ wie vorstehend definiert ist und Hal F, Cl oder Br sind,
ausgewählten Substanz.

**10.** Verwendung einer Zusammensetzung der Formel I oder ihrer Additionssalze nach Anspruch 1 zur Bestimmung des Faktors Xa.

**11.** Verfahren zur Bestimmung des Faktors Xa,
**dadurch gekennzeichnet,** daß
ein wäßriges biologisches Milieu mit einer geeigneten Menge der Zusammensetzung der Formel I und ihrer Additionssalze nach Anspruch 1 in Kontakt gebracht wird und eine Testprobe auf ihre Empfindlichkeit zum Gehalt des Faktors Xa überprüft wird.

**12.** Notwendige zur Bestimmung des Faktors Xa bestimmte Dosierung,
**dadurch gekennzeichnet,** daß
sie mindestens eine aus den Zusammensetzungen der Formel I und ihren Additionssalzen nach Anspruch 1 ausgewählte Peptidzusammensetzung und gegebenenfalls eine geeichte Probe des Faktors Xa und eines verdünnten Puffermilieus enthält.